# EUROPEAN PATENT SPECIFICATION

(11) **EP 2 073 724 B1**
(45) Date of publication and mention of the grant of the patent: **20.04.2011**
(21) Application number: 07826302.7
(22) Date of filing: 07.09.2007
(51) Int. Cl.: A61B 17/11

(54) **NEURAL GUIDE**
NEURALE FÜHRUNG
GUIDE NEURAL

(30) Priority: 08.09.2006 IT RM20060474
(43) Date of publication of application: 01.07.2009
(73) Proprietor: Universita' Cattolica Del Sacro Cuore, 20123 Milano Mi (IT)
(72) Inventor: CATALANO, Francesco, I-00189 Roma (IT); MEROLLI, Antonio, I-00189 Roma (IT)
(74) Representative: Papa, Elisabetta
(86) International application number: PCT/IB2007/053612
(87) International publication number: WO 2008/029373

(56) References cited:
- EP-A- 0 370 917
- WO-A-92/10975
- WO-A-02/096300
- DE-A1- 19 860 304
- US-A- 4 669 474
- US-A- 4 778 467
- MEYER J-U ET AL: "PERFORATED SILICON DICES WITH INTEGRATED NERVE GUIDANCE CHANNELS FOR INTERFACING PERIPHERAL NERVES" PROCEEDINGS OF THE WORKSHOP ON MICRO ELECTRICAL MECHANICAL SYSTEMS. (MEMS). AMSTERDAM, JAN. 29 - FEB. 2, 1995, NEW YORK, IEEE, US, vol. WORKSHOP 8, 29 January 1995 (1995-01-29), pages 358-361, XP000555297 ISBN: 0-7803-2504-4

## Description

The present invention refers to a neural guide, or neuroguide, i.e. to a device apt to promote regeneration of a nerve, in particular a peripheral nerve, therethrough.

In the last few years there has taken hold a technique for regenerating nerves, mostly peripheral ones, based on the use of tubular structures commonly referred to as neural guides or "neuroguides". According to such a technique, the two torn or severed nerve stumps are arranged in correspondence of the longitudinal ends of the neuroguide and inserted into the internal lumen of the latter. Nerve portions adjacent to the stumps are then sutured with thread and/or fibrin glue to the neuroguide. However, to date this surgical technique entails remarkable drawbacks, risking to thwart its clinical potential.

First of all, stump insertion into the guide proves difficult, since a perfect matching of diameters between nerve branch and guide lumen is not always attained. Moreover, this operation has to be carried out without the option of a visual checking, as evidently the internal lumen of the guide itself is not visible, and anyhow not accessible from the outside.

Moreover, thread suturing on the guide is invasive, as it entails the surgical lesion of the epineurium and the entailed chance of undesired fibroblast migration that may hinder nerve regeneration. From this standpoint, gluing techniques - both current and yet to be proposed ones - should constitute an actual progress; yet, a perfect carrying out thereof in an actual surgical field is difficult as in this case as well there can be had no visual access to the internal lumen of the guide to check for correct glue distribution.

Again, the plurality of anatomical applications would require a corresponding plurality of guide diameters to allow an optimal carrying out of the technique and avoid slacks or friction/forcing of the nerve into the guide itself, yet evidently this is not compatible with the costs of production lines for tubular guides.

US 4,669,474 discloses a medical device for the sutureless repair of lacerated nerves, comprising longitudinally-openable tubes.

WO 02/10975 discloses a medical anastomosis sling for use in the repair or regeneration of nerves, which sling can be wrapped around the nerve ends.

US 4,778,467 discloses a tubular prosthesis for promoting nerve regeneration, which prosthesis has a longitudinal slit allowing its opening.

Therefore, the technical problem set and solved by the present invention is to provide a neural guide allowing to overcome the drawbacks mentioned above with reference to the known art.

Such a problem is solved by a neural guide according to claim 1.

Preferred features of the present invention are set forth in the dependent claims thereof.

The present invention provides several relevant advantages. The main advantage lies in that the provision of a guide structure having two members that can be spaced the one from the other allows the surgeon to insert in an extremely simple and rapid manner the neural ends into the guide, visually checking all insertion steps. Analogously, the surgeon may also visually check step-by step the correct making of any gluing points.

Moreover, such advantages allow to overcome the drawback linked to the need of different diameters of the neuroguide, as even with a lower number of diameters the nerve insertion and locking technique may be optimally carried out or, vice versa, the production of a high number of diameters will be less complicated and expensive at an industrial level.

Other advantages, features and the operation modes of the present invention will be made apparent from the following detailed description of some embodiments thereof, given by way of example and not for limitative purposes. Reference will be made to the figures of the annexed drawings, wherein:
- Figure 1 shows a perspective view of a preferred embodiment of the neural guide according to the present invention;
- Figures 2 to 4 show each a perspective view of the neural guide of Figure 1, during a respective step of the surgical use thereof; and
- Figures 5 and 6 show partially see-through perspective views of the neural guide of Figure 1; Figure 5 illustrates by way of example a respective phase of the regeneration of a nerve housed into the guide itself, and Figure 6 illustrates the gap that the guide reserves to nerve regeneration.

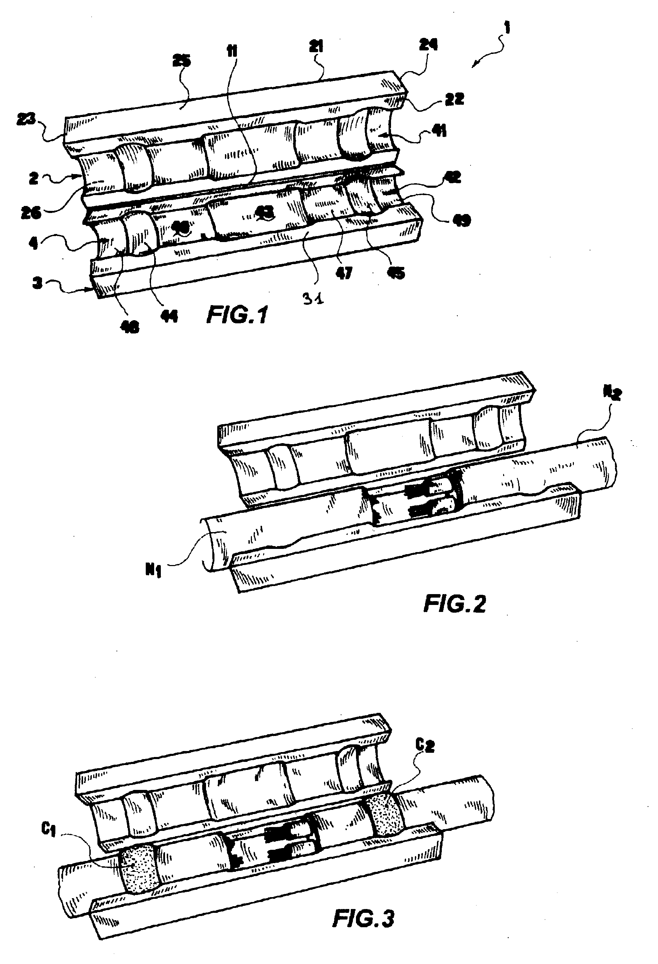

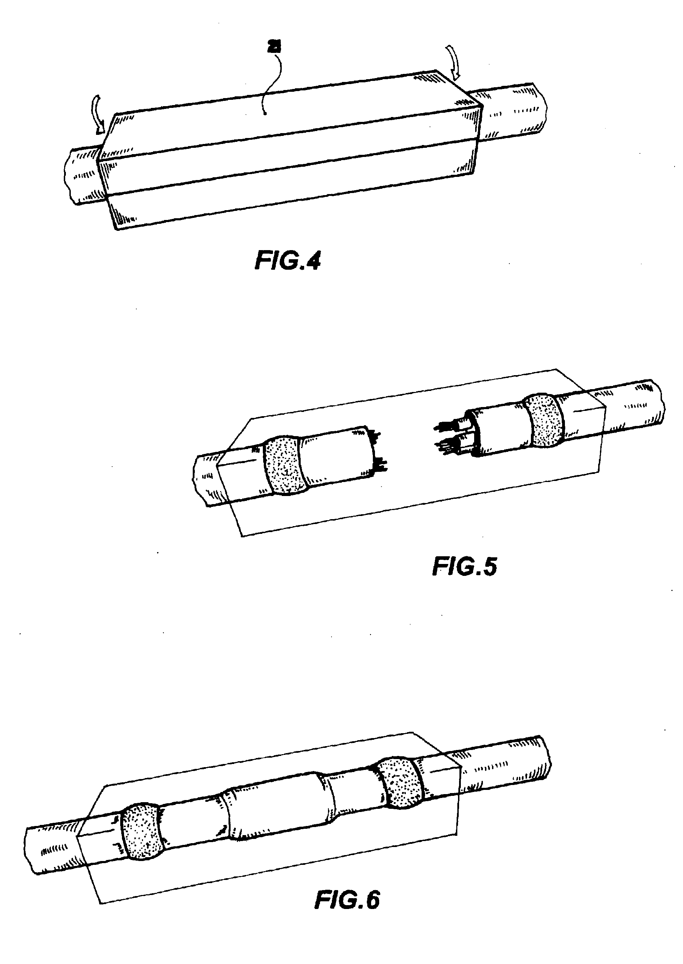

Referring initially to Figures 1 to 4, a neural guide according to a preferred embodiment of the invention is generally denoted by 1.

In the present example the neuroguide 1 has a substantially box-like structure and is formed by a first and a second member, 2 and 3 respectively, closeable the one on the other and in particular rotatably connected to each other in correspondence of a longitudinal edge 11 of the neuroguide 1 itself.

According to a preferred variant embodiment, such two members 2 and 3 closeable the one on the other are completely detachable the one from the other, instead of being permanently and/or rotatably connected. In that case - and actually also in the embodiment providing a connection of the two members 2 and 3 - the device preferably comprises restraining means, like, e.g., one or more pins and respective seats, to mutually lock the two members in a closed configuration. Said variant in which the two members 2 and 3 are completely detachable is advantageous from a productive standpoint.

The first member 2 is substantially parallelepiped-shaped, comprising a top wall 21 and a bottom wall 22, parallel and having wider extension, a pair of parallel side walls 23 and 24, a front wall 25 and a rear wall 26 parallel to the front wall 25.

In correspondence of the bottom wall 22, the member 2 defines a first semi-seat 41 with a substantially semi-cylindrical geometry developing along the entire longitudinal extension of the bottom wall 22 itself.

The second member 3 has a shape analogous to that of the first member, defining, in correspondence of a top wall 31 thereof facing on the bottom wall 22 of the first member 2, a second longitudinal half-seat 42.

Therefore, the overall configuration is such that, when the first member 2 is closed on the second member 3, the two half-seats 41 and 42 lie facing the one onto the other forming an elongate longitudinal seat 4 having a substantially cylindrical geometry and extending across the neuroguide 1 itself. Therefore, in such a closed configuration the neuroguide 1 has a tubular structure and a substantially parallelepiped-shaped external shape.

Hence, the overall configuration of each of the two members 2 and 3 may be defined as a substantially half-shell one.

Analyzing now in more detail the shape of the seat 4, this has a widened regeneration section 43 apt to promote just nerve regeneration therethrough. Such a section 43 has a substantially cylindrical geometry and is centrally arranged. According to a preferred embodiment, said section has a substantially ellipsoidal geometry. Moreover, the seat 4 has two side sections, 44 and 45 respectively, them also having a widened section and each having a substantially spherical geometry. Each of the sections 44 and 45 is apt to receive an adhesive for fixing the nerve stump therein. With regard to the remaining sections of the seat 4, this has two substantially cylindrical sections, 46 and 47 respectively, each arranged in correspondence of a respective side of the central regeneration section 43 and interposed between the latter and a respective fixing seat 44, 45, and two further substantially cylindrical sections, 48 and 49 respectively, each arranged sideways to a respective fixing section 44, 45 and extending in correspondence of a respective longitudinal end of the seat 4 itself.

Advantageously, among its different sections 43-49 the seat 4 may have one or more transition sections having rounded corners so as not to injure the nerve housed into the seat 4 itself.

The different sections of the seat 4 may be obtained with known machining techniques, e.g. by microetching.

Of course, the neuroguide 1 is made of a biocompatible and optionally non-permeable material.

The operation mode of the neuroguide 1 will now be illustrated in detail with reference to Figures 2 to 4.

As mentioned above, the neural guide 1 is apt to house into the seat 4 a proximal nerve stump and a distal nerve stump, exemplarily depicted and denoted by N1 and N2 in Figure 2, to allow their regeneration therethrough.

With a suitable distal closure the neuroguide of the invention may be used as a blind-ended or blind guide for regeneration of the sole proximal stump.

As it is shown in Figure 2, for nerve stump insertion the neuroguide 1 is brought into an open first operating configuration, shown in Figures 2 and 3, in which the two members 2 and 3 are rotated with respect to each other (or separate in the above-mentioned variant embodiment) to move them away and allow access to said seat 4, and in particular to the half-seat 42 of the second member 3. Then, the two stumps are inserted from opposite sides of the seat 4, so that their ends be facing each other in correspondence of the regeneration section 43.

Once such an insertion has been carried out, as shown in Figure 3 the nerve stumps are locked into the seat 4 by two gluing points, obtained each in correspondence of a respective fixing section 44, 45 of the seat 4 and denoted by C1 and C2. By now, it will have been better appreciated that the widened and substantially spherical shape of said sections 44 and 45 allows to optimally make said gluing points.

Moreover, to the surgeon it is offered the option of filling the seat 4 by utilizing means going from a mere saline to self-assembling peptides.

Then, the neuroguide 1 may be brought to a second closed configuration in which members 2 and 3 are rotated so as to abut to each other, housing nerve stumps therebetween.

It will be understood that the present invention is susceptible of several embodiments alternative to the hereto-described one, some of which will briefly be illustrated hereinafter with reference to the sole aspects differentiating it from the hereto-considered first embodiment.

First of all, the neuroguide may be configured so as to receive a single nerve stump, therefore being blind, as already mentioned above.

## Claims

1. A neural guide (1) apt to receive at least one nerve stump (N1, N2) to allow its regeneration therethrough, comprising a first (2) and a second (3) member defining an internal longitudinal seat (4) of the guide itself (1) and movable with respect to each other so that the guide (1) may assume a first open configuration in which said first (2) and second (3) member are moved away to allow insertion of the nerve stump into said seat (4), and a second closed configuration, in which said first (2) and second (3) member are neared to each other, closing said seat (4),
**characterised in that** said longitudinal seat (4) has at least one widened fixing section (44, 45), apt to receive an adhesive to lock the nerve stump thereinto..

2. The neural guide (1) according to claim 1, having a substantially box-like structure.

3. The neural guide (1) according to claim 1 or 2, wherein said longitudinal seat (4) extends across the guide (1) itself, the latter having, in said closed configuration, a substantially tubular structure.

4. The neural guide (1) according to claim 1 or 2, wherein said longitudinal seat (4) is blind, opening only in correspondence of a longitudinal end of the guide (1) itself

5. The neural guide (1) according to any one of the preceding claims, wherein said first (2) and second (3) member are each substantially in the form of a half shell.

6. The neural guide (1) according to any one of the preceding claims, wherein said first (2) and second (3) member are rotatably connected to each other.

7. The neural guide (1) according to any one of the preceding claims, wherein said first (2) and second (3) member are completely detachable from each other.

8. The neural guide (1) according to any one of the preceding claims, wherein said first (2) and second (3) member have each a substantially parallelepiped shape.

9. The neural guide (1) according to any one of the preceding claims, wherein said longitudinal seat (4) has at least mostly a substantially cylindrical geometry.

10. The neural guide (1) according to any of the preceding claims, wherein said seat (4) has a pair of widened fixing sections (44, 45), each apt to receive an adhesive to lock the or a respective nerve stump thereinto.

11. The neural guide (1) according to any of the preceding claims, wherein said widened fixing section or sections (44, 45) has/have a substantially spherical geometry.

12. The neural guide (1) according to any one of the preceding claims, wherein said widened fixing section or sections (44, 45) is/are arranged sideways on the seat (4) itself.

13. The neural guide (1) according to any one of the preceding claims, wherein said longitudinal seat (4) has a widened regeneration section (43) apt to promote nerve regeneration therethrough.

14. The neural guide (1) according to claim 13, wherein said widened regeneration section (43) has a substantially cylindrical geometry.

15. The neural guide (1) according to claim 13, wherein said widened regeneration section (43) has a substantially ellipsoidal geometry.

16. The neural guide (1) according to any one of the claims 13 to 15, wherein said widened regeneration section (43) is arranged in a substantially central position on the seat (4) itself.

17. The neural guide (1) according to any one of the claims 13 to 16, wherein said widened regeneration section (43) has a surface whose topography is made by microetching techniques.

18. The neural guide (1) according to any one of the claims 13 to 17, wherein into said widened regeneration section (43) it is possible to house various preparations apt to aid nerve regeneration.

19. The neural guide (1) according to any one of the preceding claims, which is apt to receive a pair of nerve stumps (N1, N2), insertable from opposite sides of said longitudinal seat (4).

## Patentansprüche

1. Nervenführung (1), die geeignet ist, wenigstens einen Nervenstumpf (N1, N2) zu empfangen, um in sich dessen Regeneration zu gestatten, mit einem ersten und einem zweiten Teil (2, 3), die einen inneren Längssitz (4) der Führung (1) selbst begrenzen und in Bezug aufeinander beweglich sind, so dass die Führung (1) eine erste, offene Konfiguration annehmen kann, in welcher das erste und das zweite Teil (2, 3) voneinander wegbewegt sind, um das Einführen des Nervenstumpfes in den Sitz (4) zu gestatten, und eine zweite, geschlossene Konfiguration, in welcher das erste und das zweite Teil (2, 3) einander genähert sind, um den Sitz (4) verschließen, **dadurch gekennzeichnet, dass** der Längssitz (4) wenigstens einen verbreiterten Fixierungsabschnitt (44, 45) hat, der geeignet ist, einen Klebstoff zu empfangen, um den Nervenstumpf darin zu arretieren.

2. Nervenführung (1) nach Anspruch 1, mit einem im Wesentlichen kastenartigen Aufbau.

3. Nervenführung (1) nach Anspruch 1 oder 2, wobei sich der Längssitz (4) über die Führung (1) selbst erstreckt und wobei letztere in der geschlossenen Konfiguration einen im Wesentlichen rohrförmigen Aufbau hat.

4. Nervenführung (1) nach Anspruch 1 oder 2, wobei der Längssitz (4) unsichtbar ist und nur jeweils an einem Längsende der Führung (1) selbst offen ist.

5. Nervenführung (1) nach einem der vorhergehenden Ansprüche, wobei das erste und das zweite Teil (2, 3) jeweils im Wesentlichen die Form einer Halbschale haben.

6. Nervenführung (1) nach einem der vorhergehenden Ansprüche, wobei das erste und das zweite Teil (2, 3) drehbar miteinander verbunden sind.

7. Nervenführung (1) nach einem der vorhergehenden Ansprüche, wobei das erste und das zweite Teil (2, 3) vollständig voneinander lösbar sind.

8. Nervenführung (1) nach einem der vorhergehenden Ansprüche, wobei das erste und das zweite Teil (2, 3) jeweils im Wesentlichen die Form eines Parallelepipeds haben.

9. Nervenführung (1) nach einem der vorhergehenden Ansprüche, wobei der Längssitz (4) wenigstens überwiegend eine im Wesentlichen zylindrische Geometrie hat.

10. Nervenführung (1) nach einem der vorhergehenden Ansprüche, wobei der Sitz (4) ein Paar verbreiterte Fixierungsabschnitte (44, 45) hat, von denen jeder geeignet ist, einen Klebstoff zu empfangen, um in sich den Nervenstumpf oder einen der Nervenstümpfe zu arretieren.

11. Nervenführung (1) nach einem der vorhergehenden Ansprüche, wobei der verbreiterte Fixierungsabschnitt oder die verbreiterten Fixierungsabschnitte (44, 45) eine im Wesentlichen sphärische Geometrie hat/haben.

12. Nervenführung (1) nach einem der vorhergehenden Ansprüche, wobei der verbreiterte Fixierungsabschnitt oder die verbreiterten Fixierungsabschnitte (44, 45) seitlich von dem Sitz (4) selbst angeordnet ist/sind.

13. Nervenführung (1) nach einem der vorhergehenden Ansprüche, wobei der Längssitz (4) einen verbreiterten Regenerierungsabschnitt (43) hat, der geeignet ist, in sich die Nervenregenerierung zu fördern.

14. Nervenführung (1) nach Anspruch 13, wobei der verbreiterte Regenerierungsabschnitt (43) eine im Wesentlichen zylindrische Geometrie hat.

15. Nervenführung (1) nach Anspruch 13, wobei der verbreiterte Regenerierungsabschnitt (43) eine im Wesentlichen elliptische Geometrie hat.

16. Nervenführung (1) nach einem der Ansprüche 13 bis 15, wobei der verbreiterte Regenerierungsabschnitt (43) in einer im Wesentlichen zentralen Position auf dem Sitz (4) angeordnet ist.

17. Nervenführung (1) nach einem der Ansprüche 13 bis 16, wobei der verbreiterte Regenerierungsabschnitt (43) eine Oberfläche hat, deren Topografie durch Mikroätztechniken hergestellt ist.

18. Nervenführung (1) nach einem der Ansprüche 13 bis 17, wobei es möglich ist, in den verbreiterten Regenerierungsabschnitt (43) verschiedene Präparate einzubringen, die geeignet sind, bei der Nervenregenerierung zu helfen.

19. Nervenführung (1) nach einem der vorhergehenden Ansprüche, welche geeignet ist, ein Paar Nervenstümpfe (N1, N2) zu empfangen, die von entgegengesetzten Seiten des Längssitzes (4) aus einführbar sind.

## Revendications

1. Guide neural (1) apte à recevoir au moins un moignon de nerf (N1, N2) pour permettre sa régénération à travers celui-ci, comprenant un premier (2) et un deuxième (3) élément définissant un siège longitudinal interne (4) du guide lui-même (1) et mobiles l'un par rapport à l'autre, de sorte que le guide (1) peut prendre une première configuration ouverte dans laquelle lesdits premier (2) et deuxième (3) éléments sont écartés pour permettre l'insertion du moignon de nerf dans ledit siège (4), et une deuxième configuration, fermée, dans laquelle lesdits premier (2) et deuxième (3) éléments sont rapprochés l'un de l'autre et ferment ledit siège (4),
**caractérisé en ce que** ledit siège longitudinal (4) comporte au moins une section de fixation élargie (44, 45), apte à recevoir un adhésif pour y immobiliser le moignon de nerf.

2. Guide neural (1) selon la revendication 1, présentant une structure substantiellement en forme de boîte.

3. Guide neural (1) selon la revendication 1 ou 2, dans lequel ledit siège longitudinal (4) s'étend d'un bord à l'autre du guide (1) lui-même, ce dernier présentant, dans ladite configuration fermée, une structure substantiellement tubulaire.

4. Guide neural (1) selon la revendication 1 ou 2, dans lequel ledit siège longitudinal (4) est borgne, ne s'ouvrant qu'en correspondance avec une extrémité longitudinale du guide (1) lui-même.

5. Guide neural (1) selon l'une quelconque des revendications précédentes, dans lequel lesdits premier (2) et deuxième (3) éléments ont chacun sensiblement la forme d'une demi coque.

6. Guide neural (1) selon l'une quelconque des revendications précédentes, dans lequel lesdits premier (2) et deuxième (3) éléments sont connectés l'un à l'autre en rotation.

7. Guide neural (1) selon l'une quelconque des revendications précédentes, dans lequel lesdits premier (2) et deuxième (3) éléments sont complètement détachables l'un de l'autre.

8. Guide neural (1) selon l'une quelconque des revendications précédentes, dans lequel lesdits premier (2) et deuxième (3) éléments ont chacun une forme substantiellement parallélépipédique.

9. Guide neural (1) selon l'une quelconque des revendications précédentes, dans lequel ledit siège longitudinal (4) a au moins principalement une géométrie substantiellement cylindrique.

10. Guide neural (1) selon l'une quelconque des revendications précédentes, dans lequel ledit siège (4) comporte une paire de sections de fixation élargies (44, 45), aptes chacune à recevoir un adhésif pour y immobiliser le moignon de nerf ou un moignon de nerf respectif.

11. Guide neural (1) selon l'une quelconque des revendications précédentes, dans lequel ladite ou lesdites sections de fixation élargies (44, 45) a/ont une géométrie substantiellement sphérique.

12. Guide neural (1) selon l'une quelconque des revendications précédentes, dans lequel ladite ou lesdites sections de fixation élargies (44, 45) est/sont placée(s) de côté sur le siège (4) lui-même.

13. Guide neural (1) selon l'une quelconque des revendications précédentes, dans lequel ledit siège longitudinal (4) comporte une section de régénération élargie (43) apte à favoriser la régénération des nerfs à travers elle.

14. Guide neural (1) selon la revendication 13, dans lequel ladite section de régénération élargie (43) a une géométrie substantiellement cylindrique.

15. Guide neural (1) selon la revendication 13, dans lequel ladite section de régénération élargie (43) a une géométrie substantiellement ellipsoïdale.

16. Guide neural (1) selon l'une quelconque des revendications 13 à 15, dans lequel ladite section de régénération élargie (43) est placée dans une position substantiellement centrale sur le siège (4) lui-même.

17. Guide neural (1) selon l'une quelconque des revendications 13 à 16, dans lequel ladite section de régénération élargie (43) a une surface dont la topographie est réalisée par des techniques de microgravure.

18. Guide neural (1) selon l'une quelconque des revendications 13 à 17, dans lequel il est possible de loger dans ladite section de régénération élargie (43) diverses préparations aptes à favoriser la régénération des nerfs.

19. Guide neural (1) selon l'une quelconque des revendications précédentes, apte à recevoir une paire de moignons de nerfs (N1, N2) pouvant être insérés depuis les côtés opposés dudit siège longitudinal (4).
